(19) Europäisches Patentamt
European Patent Office
Office européen des brevets

(11) EP 3 170 504 B1

(12) **EUROPEAN PATENT SPECIFICATION**

(45) Date of publication and mention of the grant of the patent:
**29.08.2018 Bulletin 2018/35**

(21) Application number: **15822112.7**

(22) Date of filing: **16.07.2015**

(51) Int Cl.:
***A61K 9/127*** *(2006.01)* ***A61K 47/18*** *(2017.01)*
*A61K 31/7088* *(2006.01)*

(86) International application number:
**PCT/JP2015/070398**

(87) International publication number:
**WO 2016/010111 (21.01.2016 Gazette 2016/03)**

(54) **METHOD FOR PRODUCING LIPID PARTICLE AND NUCLEIC ACID DELIVERY CARRIER COMPRISING LIPID PARTICLE**

VERFAHREN ZUR HERSTELLUNG EINES LIPIDPARTIKELS UND NUKLEINSÄUREFREISETZUNGSTRÄGER MIT LIPIDPARTIKEL

PROCÉDÉ DE PRODUCTION DE PARTICULE LIPIDIQUE ET TRANSPORTEUR D'ADMINISTRATION D'ACIDE NUCLÉIQUE COMPRENANT LADITE PARTICULE LIPIDIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2014 JP 2014146809**

(43) Date of publication of application:
**24.05.2017 Bulletin 2017/21**

(73) Proprietor: **Fujifilm Corporation**
**Minato-ku**
**Tokyo 106-8620 (JP)**

(72) Inventors:
- **NISHIKAWA, Naoyuki**
**Ashigarakami-gun**
**Kanagawa 258-8577 (JP)**
- **SUGIYAMA, Susumu**
**Ashigarakami-gun**
**Kanagawa 258-8577 (JP)**
- **SEKIGUCHI, Takahiro**
**Ashigarakami-gun**
**Kanagawa 258-8577 (JP)**
- **ONO, Makoto**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**WO-A1-97/49723**
**WO-A1-97/49723**
**JP-A- H10 504 021**
**JP-A- 2002 501 511**
**JP-A- 2013 517 265**
**JP-A- 2013 537 518**
**US-A- 6 103 216**
**US-A1- 2012 114 831**
**US-A1- 2012 294 944**
**US-A1- 2013 171 241**

- **MASATO TANAKA KNOW-HOW IN PREPARATION OF NANO/MICRO-CAPSULES 06 May 2008, pages 24 - 25, XP008183717**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates to a method for producing lipid particles and an application of the lipid particles, and suitably relates to a method for producing lipid particles which is useful for delivering nucleic acids into cells, and an application of the lipid particles.

### Background Art

Nucleic acid medicines have been disclosed as next-generation pharmaceutical products since an action mechanism thereof with respect to diseases is obvious and there are few adverse reactions. For example, nucleic acid medicines in which RNA interference (RNAi) is used can cause decomposition of mRNA of a target gene existing in a cell and can inhibit expression of the target gene. As a result, it is possible to reduce or treat disease symptoms caused by abnormal expression of a specific gene or a gene group. Nucleic acids, for example, siRNA, are used in such nucleic acid medicines in which RNA interference is used. However, it is necessary to deliver nucleic acids into cells in order to exhibit a function with these nucleic acids.
In general, a carrier (vector) is used in methods for effectively delivering nucleic acids into cells. Since nucleic acids are anionic, a cationic liposome (Non-Patent Document 1) in which a cationic lipid is used as the carrier (vector), an amphoteric liposome (Patent Documents 1 and 2) which has both cationic properties and anionic properties, and the like have been reviewed.
The Bangham method is known as the most usual method for producing a liposome. The Bangham method is a method for obtaining a liposome as follows. A phospholipid is dissolved in an organic solvent such as chloroform in a container and the organic solvent is subsequently evaporated to produce a thin lipid film on the inner surface of the container. Thereafter, the thin film is made to swell by adding water to the thin film and the container is shaken. Patent Document 3 discloses a method for producing liposomes encapsulating nucleic acid, the method comprising dissolution of lipids in ethanol (example 7). In addition, methods such as an organic solvent extraction method, a surfactant-removing method, and a freezing and thawing method are known.

### Prior Art Documents

### Patent Documents

Patent Document 1: JP2011-21026A
Patent Document 2: JP2005-517739A
Patent Document 3: US 2012/0114831A1

### Non-Patent Document

Non-Patent Document 1: Gene Therapy, Vol. 6, p. 271, 1999

## SUMMARY OF THE INVENTION

### Object to be solved by the Invention

There is the inclusion rate as an important index for evaluating a carrier (vector). The inclusion rate is an index indicating what percentage of hydrophilic substances which have been added for being held in an internal water phase while producing a carrier (vector) is held in the internal water phase.
In a method for producing a carrier (vector) in the related art, it is insufficient to obtain a carrier (vector) which can, for example, hold nucleic acids at a high inclusion rate.
An object of the present invention is to provide a method for producing lipid particles which can hold nucleic acids or the like at a high inclusion rate, and a nucleic acid delivery carrier having the lipid particles.

### Means for Solving the Object

The present inventors have conducted extensive studies in order to solve the above-described problem. As a

result, they have found that it is possible to provide a production method as defined in the claim 1 for producing lipid particles by which the above-described problem is solved, and have completed the present invention.
That is, means for solving the problem is as follows.

[1] A method for producing lipid particles including nucleic acids, the method comprising the following steps of (1) to (4):

(1) a step of heating an oil phase containing a phospholipid, ethanol, and ethyl acetate;
(2) a step of mixing an oil phase prepared in step (1) with a water phase containing nucleic acids, wherein the mass ratio of mixing the water phase with the oil phase is 1.6:1.0 to 1.1:1.0;
(3) a step of cooling the mixed liquid which contains the oil phase and the water phase and has been obtained in step (2), and crystallizing lipid particles; and
(4) a step of removing ethanol and ethyl acetate from the mixed liquid which contains the oil phase and the water phase and has been obtained in step (3).

[2] The method for producing lipid particles according to [1],wherein, in step (1), the oil phase further contains a compound having at least one amino group and at least one imidazoyl group.
[3] The method for producing lipid particles according to [2],wherein the compound having at least one amino group and at least one imidazoyl group is a compound represented by General Formula (1),

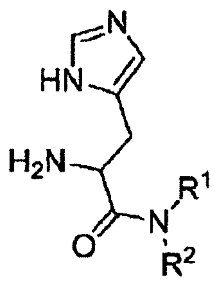
(1)

In the formula, $R^1$ and $R^2$ are the same as or different from each other and are substituents selected from an alkyl group having 10 to 22 carbon atoms, an alkyloxyalkylene group having 10 to 22 carbon atoms, an alkanoyloxyalkylene group having 10 to 22 carbon atoms, and an alkyloxycarbonylalkylene group having 10 to 22 carbon atoms.
[4] The method for producing lipid particles according to any one of [1] to [3], wherein, in step (1), the oil phase containing a phospholipid, ethanol, and ethyl acetate is heated at 40°C to 70°C.
[5] The method for producing lipid particles according to any one of [1] to [4], wherein, in step (3), the mixed liquid containing an oil phase and a water phase is cooled at 10°C to 30°C.

Advantageous effect of the invention

According to the method for producing lipid particles of the present invention, it is possible to provide a method for producing lipid particles which can hold nucleic acids or the like at a high inclusion rate, and a nucleic acid delivery carrier having the lipid particles.

BRIEF DESCRIPTION OF THE DRAWINGS

Fig. 1 is a view of $^1$H-NMR of a compound A in a synthesis example.
Fig. 2 is a view of an MS spectrum of the compound A in the synthesis example.

DESCRIPTION OF THE EMBODIMENTS OF THE INVENTION

Hereinafter, the present invention will be described in detail.
"to" in the present specification indicates a range respectively including numerical values described before and after "to" as minimum values and maximum values.

(1) Method for Producing Lipid Particles

Production of lipid particles of the present invention is a method for producing (hereinafter, in some cases, referred to as a production method of the present invention) lipid particles including nucleic acids, the method comprising the steps as defined in the claim 1.

The production method of the present invention is a method for forming particles in which coacervation is used. Coacervation is a phenomenon of separating two phases of a liquid phase which is rich in colloid and a liquid phase which is lack in colloid, from each other in a case where other substances are added to hydrocolloid, a poor solvent is used for diluting, or the pH is changed.

In the production method of the present invention, a kind of coacervation phenomenon occurs such that an oil phase containing a phospholipid, ethanol, and ethyl acetate is heated, and a water phase, which contains nucleic acids, and the obtained oil phase are mixed with each other, and then, the mixture is cooled over time. A liposome in which nucleic acids are held at a high inclusion rate can be obtained through self-organization such that the phospholipid contains nucleic acids.

[Step (1)]

The production method of the present invention includes step (1): a step of heating an oil phase containing a phospholipid, an alcohol, and an ester (the oil phase means an oily component contained in a composition obtained by mixing a phospholipid, ethanol, and ethyl acetate).

The phospholipid used in the present invention is not particularly limited. Examples thereof include natural phospholipids such as phosphatidylcholine, phosphatidylethanolamine, phosphatidylserine, phosphatidylinositol, lysophosphatidylcholine, sphingomyelin, egg yolk lecithin, and soybean lecithin; a hydrogenated phospholipid in which an unsaturated carbon chain of a naturally derived phospholipid is saturated by hydrogen; and a synthetic phospholipid in which modification is applied to a naturally derived phospholipid through synthesis, and these may be used singly or two or more kinds thereof may be combined.

The total amount of phospholipid in the present invention with respect to nucleic acids is preferably 10: 1 to 5000:1 and more preferably 100:1 to 1000:1 in a molar ratio.

As an alcohol used in the present invention, ethanol is used from the viewpoint of polarity.

As ester used in the present invention, an ethyl acetate is used from the viewpoint of polarity or lipophilicity.

The ratio of an alcohol to an ester in the present invention is preferably 90:10 to 10:90, more preferably 80:20 to 30:70, and still more preferably 70:30 to 40:60 in a capacity ratio.

In the production method of the present invention, in step (1), it is preferable that the oil phase further contain a compound having at least one amino group and at least one imidazoyl group (preferably as a lipid).

The formulation amount in a case of using the compound having at least one amino group and at least one imidazoyl group with respect to the total amount of lipid is preferably 10 mol% to 70 mol%, more preferably 15 mol% to 60 mol%, and still more preferably 20 mol% to 50 mol%.

The compound having at least one amino group and at least one imidazoyl group used in the present invention is not particularly limited, but it is preferable to use, for example, a compound represented by General Formula (1).

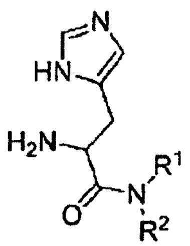

(1)

In the formula, $R^1$ and $R^2$ are the same as or different from each other and are substituents selected from an alkyl group having 10 to 22 carbon atoms, an alkyloxyalkylene group having 10 to 22 carbon atoms, an alkanoyloxyalkylene group having 10 to 22 carbon atoms, and an alkyloxycarbonylalkylene group having 10 to 22 carbon atoms.

$R^1$ and $R^2$ are the same as or different from each other, and are more preferably an alkyl group having 10 to 22 carbon atoms and still more preferably an alkyl group having 14 to 20 carbon atoms. In addition, the alkyl group may have a double bond.

The compound having at least one amino group and at least one imidazoyl group, which is used in the production method of the present invention and is represented by General Formula (1) is not particularly limited, and can be synthesized through, for example, the following method.

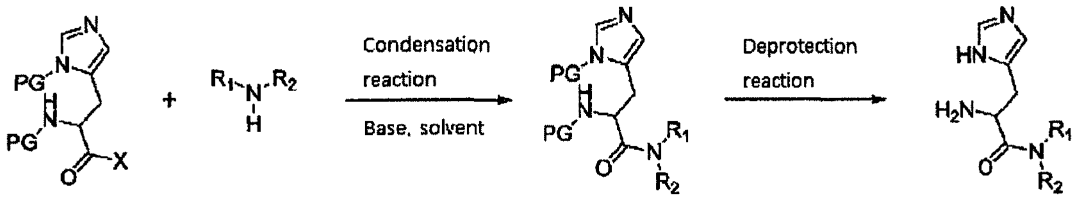


(A) (B) (C) (1)

In the formula, PG represents a protective group and X represents a leaving group constituting an active ester. $R^1$ and $R^2$ are the same as the description above.

That is, after obtaining a compound (C) by reacting an amine derivative (B) with an active ester (A) of histidine protected by a suitable protective group, in the presence of base, it is possible to synthesize the compound represented by General Formula (1) through a suitable deprotection method.

Here, examples of the protective group which can be used in the active ester (A) of histidine include a protective group disclosed in W. Greene et al., Protective Groups in Organic Synthesis 4th edition, pages 255 to 265, 2007, John Wiley & Sons, INC. Specifically, preferred examples thereof include a tert-butoxycarbonyl group (Boc group) and a benzyloxycarbonyl group (Z group).

Examples of the active ester which can be used include a phenyl ester, a trifluorophenyl ester, a pentaphenyl ester, and a hydroxysuccinimide ester. A hydroxysuccinimide ester is preferable from the viewpoint of raw material-obtaining properties or stability.

Examples of the base which can be used include an inorganic base and an organic base. Examples of the inorganic base include sodium hydrogen carbonate or sodium carbonate, and examples of the organic base include triethylamine and diisopropylamine. As the base to be used, it is preferable to use a suitable base using a protective group of the active ester (A) of histidine used for a reaction.

The solvent which can be used is not particularly limited, and in general, it is possible to use an organic solvent. Specific examples thereof include an ether-based solvent, an ester-based solvent, an amide-based solvent, and a halogen-based solvent, and preferred examples thereof include ether-based solvents such as tetrahydrofuran and halogen-based solvents such as dichloromethane and chloroform.

Examples of the deprotection reaction which can be used include a method disclosed in W. Greene et al., Protective Groups in Organic Synthesis 4th edition, pages 255 to 265, 2007, John Wiley & Sons, INC.

As the compound having at least one amino group and at least one imidazoyl group which is used in the production method of the present invention, it is preferable to use the compound (2-amino-N,N-dihexadecyl-3-(1H-imidazol-5-yl)propanamide) [also referred to a compound A in the present specification] represented by Formula (2).

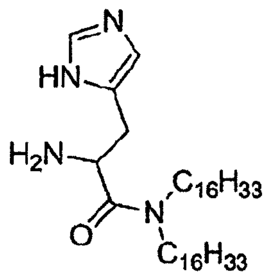
(2)

In the production method of the present invention, it is possible to hold nucleic acids at a high inclusion rate using the compound represented by Formula (2) and to obtain lipid particles excellent in releasability of nucleic acids in a target cell.

In step (1) of the present invention, the temperature in a case of heating the oil phase containing a phospholipid, an alcohol, and an ester is preferably 40°C to 70°C, more preferably 45°C to 65°C, and still more preferably 50°C to 60°C.

In addition, the heating time is not particularly limited as long as it is possible to check that the temperature of the entirety of a liquid uniformly becomes a desired temperature.

[Other Components]

The oil phase of the present invention can contain other components as necessary in addition to phospholipids, ethanol, and ethyl acetate in a range of not impairing the effect of the present invention.

(Sterol)

In the production method of the present invention, the oil phase may contain sterol. In the present invention, it is possible to obtain a stabilization effect of lipid particles by reducing membrane fluidity due to the inclusion of sterol in the oil phase.
Sterol is not particularly limited, but examples thereof include cholesterol, phytosterol (sitosterol, stigmasterol, fucosterol, spinasterol, and brassicasterol), ergosterol, cholestanone, cholestenone, coprostanol, cholesteryl-2'-hydroxyethyl ether, and cholesteryl-4'-hydroxybutyl ether. Among these, cholesterol is preferable.
In the present invention, the formulation amount of sterol with respect to the total amount of lipid is preferably 10 mol% to 60 mol%, more preferably 20 mol% to 55 mol%, and still more preferably 25 mol% to 50 mol%.

(Lipid Having Polyethylene Glycol Chain)

In the production method of the present invention, the oil phase may contain a lipid having a polyethylene glycol chain (hereinafter, referred to as a "PEG chain"). In the present invention, it is possible to obtain a dispersion stabilization effect of lipid particles due to the inclusion of the lipid having a PEG chain in the oil phase.
The lipid having a PEG chain is not particularly limited, and examples thereof include PEG-modified phosphoethanolamine, diacylglycerol PEG derivatives, dialkyl glycerol PEG derivatives, cholesterol PEG derivatives, and ceramide PEG derivatives. Among these, PEG-modified phosphoethanolamine is preferable.
The weight-average molecular weight of the PEG chain is preferably 500 to 5000 and more preferably 750 to 2000.
The PEG chain may be branched or may have a substituent such as a hydroxymethyl group.
In the present invention, the formulation amount of lipid having a PEG chain with respect to the total amount of lipid is preferably 0.5 mol% to 12 mol%, more preferably 2 mol% to 10 mol%, and still more preferably 4 mol% to 8 mol%.

[Step (2)]

The production method of the present invention includes step (2): a step of mixing an oil phase prepared in step (1) with a water phase containing nucleic acids.

[Nucleic acid]

Well-known nucleic acids in an arbitrary form are included in the nucleic acids used in the present invention. Specific examples of the nucleic acids include general RNA, DNA, and derivatives thereof. Single-stranded DNA or RNA may be used, double stranded DNA or RNA may be used, and DNA-RNA hybrid may be used. Specific examples of the nucleic acids used in the present invention include antisense DNA, antisense RNA, DNA enzyme, ribozyme, siRNA, shRNA, miRNA, aiRNA, piRNA, decoy nucleic acids, and aptamer. siRNA, miRNA, aiRNA, antisense DNA, and antisense RNA are preferably used as the nucleic acids used in the present invention.
The nucleic acids used in the present invention are not limited to be in a natural type, and may be in a non-natural type, in which at least a part of sugar or phosphate backbone or the like constituting nucleotide is modified in order to improve stability, such as nuclease resistance, in a living body.
Examples of the non-natural nucleic acids in which the sugar portion is modified include 2'-O-methyl RNA, 2'-O-(2-methoxy)ethyl RNA, 2'-deoxy-2'-fluoroarabino nucleic acids, and cross-linked nucleic acids (LNA/BNA). In addition, other examples of the non-natural nucleic acids include peptide nucleic acids (PNA) in which the sugar portion is replaced with peptide, morpholino nucleic acids in which the sugar portion is replaced with morpholino.
Examples of the non-natural nucleic acids in which a phosphate backbone is modified include phosphorothioate body and phosphorodithioate body.
A water phase (water phase means an aqueous component) in the production method of the present invention can be obtained by, for example, making nucleic acids be dissolved in an aqueous component such as water.
In step (2) of the present invention, the water phase and the oil phase which has been obtained in step (1) are mixed with each other. The ratio (mass ratio) of mixing the water phase with the oil phase is 1.6:1.0 to 1.1:1.0.
The temperature in a case of mixing the water phase and the oil phase is preferably 40°C to 70°C, more preferably 45°C to 65°C, and still more preferably 50°C to 60°C.
In addition, any mixing time may be selected as long as it is possible to confirm that the entirety of a liquid becomes uniform, and the mixing time is not particularly limited. In addition, any heating time may be selected as long as it is possible to confirm that the temperature of the entirety of a liquid uniformly becomes a desired temperature, and the heating time is not particularly limited.

[Step (3)]

The production method of the present invention includes step (3): a step of cooling the oil phase-water phase mixed liquid and has been obtained in step (2), and crystallizing lipid particles.
In step (3) of the present invention, the cooling conditions of the oil phase-water phase mixed liquid is preferably 10°C to 30°C and more preferably 15°C to 25°C.
In addition, the cooling time is not particularly limited, but the cooling rate is preferably lower than or equal to -3°C/minute.

[Step (4)]

The production method of the present invention includes step (4): a step of removing the alcohol and the ester from the oil phase-water phase mixed liquid and has been obtained in step (3).
In step (4) of the present invention, the method for removing ethanol and ethyl acetate from the oil phase-water phase mixed liquid is not particularly limited, and ethanol and ethyl acetate can be removed therefrom through a general technique.

[Other Steps]

Sizing can be performed on lipid particles which have been obtained through the production method of the present invention as necessary. When performing the sizing, it is possible to reduce the particle diameter by subjecting a liquid (suspension), which contains lipid particles, to an ultrasonic treatment through any one of an in-tank ultrasonic treatment and a probe ultrasonic treatment.
In addition, the lipid particles obtained through the production method of the present invention can be concentrated as necessary. When performing the concentrating, it is possible to employ various well-known methods. Examples thereof include a concentration method in which an ultrafiltration membrane is used.

(2) Regarding Lipid Particles

In the present invention, the lipid particles means particles constituted of lipids, and are not particularly limited. A liposome having a lamellar structure which is a closed endoplasmic reticulum constituted of a lipid bimolecular membrane is contained in the lipid particles of the present invention. Structures such as a multi liposome (MLV), a small unilamellar liposome (SUV), or a giant unilamellar liposome is known as the liposome, but are not particularly limited thereto. Particles, which do not have the lipid bimolecular membrane structure (lamellar structure) of the above-described liposome, but have a structure in which the inside of a particle is filled with constituent components, are also included in the lipid particles of the present invention.
The form of the lipid formation can be checked through structure analysis through electron microscopic observation or using X-rays. For example, it is possible to confirm that a lipid particle has a lipid bimolecular membrane structure (lamellar structure) like the liposome does and a structure having an internal water layer, or a structure filled with constituent components including lipids since a lipid particle has a core with high electron density in the inside of the particle without having a lipid bimolecular membrane structure (lamellar structure) like the liposome does and an internal water layer, through a method in which Cryo transmission electron microscopy observation (CryoTEM method) is used. It is also possible to check the presence and absence of the lipid bimolecular membrane structure (lamellar structure) in a lipid particle even through X-ray small angle scattering (SAXS) measurement.
The particle diameter of the lipid particles of the present invention is not particularly limited, but is preferably 10 to 1000 nm, more preferably 50 to 500 nm, and still more preferably 75 to 350 nm. The particle diameter of the lipid particles can be measured through a general method (for example, a dynamic light scattering method or a laser diffraction method).

(3) Use of Lipid Particle

It is possible to introduce nucleic acids or the like (for example, genes) into cells by introducing lipid particles into the cell in vitro as an example of lipid particles obtained through the production method of the present invention. In addition, in a case where the lipid particles obtained through the production method of the present invention contain nucleic acids having a medicinal use, it is possible to administer the lipid particles into a living body as nucleic acid medicines.
In a case of using the lipid particles obtained through the production method of the present invention as nucleic acid medicines, it is possible to administer the lipid particles of the present invention into a living body singly or after

being mixed with a dose vehicle (for example, a physiological saline or a phosphate buffer solution) which is pharmaceutically acceptable.

The concentration of lipid particles in a mixture mixed with a carrier which is pharmaceutically acceptable is not particularly limited, and can be generally set to 0.05 mass% to 90 mass%. In addition, other additives, for example, a pH adjusting and buffering agent or an osmotic pressure-controlling agent, which are pharmaceutically acceptable, may be added to nucleic acid medicines containing the lipid particles of the present invention.

The administration route when administering nucleic acid medicines containing the lipid particles of the present invention are administered in vivo is not particularly limited, and nucleic acid medicines can be administered through an arbitrary method. Examples of the administration method include oral administration, and parenteral administration (intra-articular administration, intravenous administration, intraperitoneal administration, and muscle administration). Nucleic acid medicines containing the lipid particles of the present invention can also be administered by being directly injected into a disease site.

A dosage form of lipid particles of the present invention is not particularly limited. However, in a case of performing oral administration, the lipid particles of the present invention can be used in forms of tablets, trochiscus, capsules, pills, suspensions, and syrups by being combined with an appropriate diluting agent. In addition, antioxidants, buffering agents, bacteriostats, and additives such as isotonic sterile injections, suspending agents, solubilizing agents, thickening agents, stabilizers, or preservatives can be appropriately included in pharmaceutical preparations suitable for parenteral administration.

(4) Nucleic Acid Delivery Carrier

The lipid particles obtained through the production method of the present invention can hold nucleic acids at a high inclusion rate, and therefore, the lipid particles are significantly useful as nucleic acid delivery carriers. The nucleic acid delivery carriers of the present invention can introduce nucleic acids or the like into cells by, for example, being transfected into cells in vitro after mixing the obtained lipid particles with the nucleic acids or the like. In addition, the nucleic acid delivery carriers of the present invention are useful as nucleic acid delivery carriers in nucleic acid medicines.

Examples

The present invention will be described in detail using the following Examples, but the scope of the present invention is not limited to the following Examples.

In addition, in the present invention, CHOLESTEROL HP manufactured by Dishman Pharmaceuticals & Chemicals Ltd. was used as cholesterol, COATSOME MC6060 manufactured by NOF CORPORATION was used as dipalmitoylphosphatidylcholine (DPPC), SUNBRIGHT DSPE-020CN manufactured by NOF CORPORATION was used as polyethylene glycol-modified phosphoethanolamine (DSPE-PEG, PEG chain molecular weight: 2000), and COATSOME NC-50 manufactured by NOF CORPORATION was used as lecithin.

[Synthesis Example: Synthesis of Compound (Hereinafter, Compound A) Represented by Formula (2)]

First Step

23 g of dihexadecylamine and 5.52 g of triethylamine were added to 230 mL of tetrahydrofuran, 24.6 g of Boc-His(1-Boc)-OSU was added thereto while stirring the mixture, which was then stirred for one hour at room temperature and was stirred for five hours at 50°C. Thereafter, tetrahydrofuran was distilled off under reduced pressure and 450 mL of chloroform and 200 mL of water were added to the reactant. An organic layer was separately taken, sequentially washed with a saturated sodium hydrogen carbonate aqueous solution, a 10% citric acid aqueous solution, and a saturated sodium chloride aqueous solution, and dried with anhydrous magnesium sulfate to distil off a solvent under reduced pressure. The residue was purified through silica gel column chromatography (hexane / ethyl acetate = 5/1 to 3/1) and 24 g of a protected substance of an oily matter was obtained.

Boc-His(1-Boc)-OSU

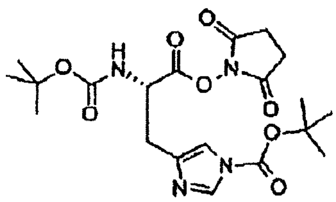

Second Step

21.7 g of the protected substance which had been obtained in the first step was added to 35 mL of trifluoroacetic acid little by little, and the mixture was stirred for 24 hours at room temperature. Thereafter, the mixture was gradually added to 600 mL of an aqueous solution containing 40 g of saturated sodium bicarbonate and was stirred for one hour. 500 mL of chloroform was added to the obtained reaction liquid, an organic layer was separately taken, sequentially washed with a saturated sodium hydrogen carbonate aqueous solution and a saturated sodium chloride aqueous solution, and dried with anhydrous magnesium sulfate to distil off a solvent under reduced pressure. The residue was purified through silica gel chromatography (chloroform / methanol = 10/1) and 11.6 g of a compound A of a colorless solid was obtained. Identification of the compound was performed through NMR and MS.

[Example 1]

(Coacervation Method)

Preparation of Oil Phase

80 mg, 20 mg, 14 mg, and 20 mg of respective L-α-dipalmitoylphosphatidylcholine, lecithin, cholesterol, N-(carbonyl-methoxypolyethylene glycol 2000)-1,2-distearoyl-sn-glycero-3-phosphoethanolamine sodium salts (hereinafter, DSPE-PEG) were measured so as to make a molar ratio of 61/15/20/4, and 0.3 mL of ethanol and 0.7 mL of an ethyl acetate were added thereto and were dissolved to obtain an oil phase.

Preparation of Nucleic Acid-Holding Lipid Particle

0.25 mL of an aqueous solution of nucleic acids, which had been obtained by dissolving 5 mg of siRNA to be described below in 0.263 mL of sterilized water, was added to the oil phase obtained in the above-described step and 1.0 mL of sterilized water was further added thereto. The mixture was heated for 10 minutes at 55°C. Thereafter, the mixture was allowed to cool at room temperature while being stirred. Subsequently, dialysis was performed at room temperature using a 100 mM histidine solution to remove an ethanol/ethyl acetate-mixed solution. The obtained solution was graded by being passed through a 0.4 μm filter using an extruder (Mini Extruder manufactured by Avanti Lipids Polar, Inc.) to obtain lipid particles holding nucleic acids.

[Example 2]

(Coacervation Method)

Preparation of 200 mM Histidine Solution

15.5 g of L-histidine was measured and was dissolved in 500 mL of sterilized water. 200 mM HCl was added thereto so as to make the pH of the mixture be 7 to obtain a 200 mM histidine solution.

Preparation of Oil Phase

37 mg, 30 mg, 33 mg, and 20 mg of respective L-α-dipalmitoylphosphatidylcholine, a compound A, cholesterol, and DSPE-PEG were measured so as to make a molar ratio of 26/26/44/4, and 0.3 mL of ethanol and 0.7 mL of an ethyl acetate were added thereto and were dissolved to obtain an oil phase.

Preparation of Nucleic Acid-Holding Lipid Particle

0.25 mL of an aqueous solution of nucleic acids, which had been obtained by dissolving 5 mg of siRNA to be

described below in 0.263 mL of sterilized water, was added to the oil phase obtained in the above-described step, 0.625 mL of the 200 mM histidine solution, and 0.375 mL of sterilized water was further added thereto, and the mixture was heated for 10 minutes at 55°C. Subsequently, dialysis was performed at room temperature using a 100 mM histidine solution to remove an ethanol/ethyl acetate-mixed solution. The obtained solution was graded by being passed through a 0.4 μm filter using an extruder (Mini Extruder manufactured by Avanti Lipids Polar, Inc.) to obtain lipid particles holding nucleic acids.

[Example 3]

(Coacervation Method)

In preparation of an oil phase, lipid particles holding nucleic acids were obtained by preparing the oil phase in the same manner as in Example 2 except that 31 mg, 31 mg, 33 mg, and 44 mg of respective L-α-dipalmitoylphosphatidylcholine, a compound A, cholesterol, and DSPE-PEG were measured so as to make a molar ratio of 26/22/44/8.

Comparative Example 1

(Bangham Method)

Preparation of Oil Phase

37 mg, 30 mg, 33 mg, and 20 mg of respective L-α-dipalmitoylphosphatidylcholine, a compound A, cholesterol, and DSPE-PEG were measured so as to make a molar ratio of 26/26/44/4, and 0.3 mL of ethanol and 0.7 mL of an ethyl acetate were added thereto and were dissolved to obtain an oil phase.

Formation of Lipid Membrane

The oil phase obtained in the above-described steps was placed in an eggplant flask and ethanol and an ethyl acetate were distilled off using an evaporator to obtain a lipid membrane.

Preparation of Nucleic Acid-Holding Lipid Particle

0.25 mL of an aqueous solution of nucleic acids, which had been obtained by dissolving 5 mg of siRNA to be described below in 0.263 mL of sterilized water, and 0.625 mL of a 200 mM histidine solution were added to the above-described eggplant flask, and 0.375 mL of sterilized water were further added thereto. The mixture was hydrated while being stirred using a Vortex mixer at 55°C. The obtained solution was graded by being passed through a 0.4 μm filter using an extruder (Mini Extruder manufactured by Avanti Lipids Polar, Inc.) to obtain lipid particles holding nucleic acids.

[Comparative Example 2]

(Coacervation Method)

In preparation of an oil phase, lipid particles holding nucleic acids were obtained by preparing the oil phase in the same manner as in Example 2 except that a mixed liquid of 0.3 mL of ethanol and 0.7 mL of an ethyl acetate was dissolved instead of 1 mL of ethanol.

[Comparative Example 3]

(Bangham Method)

Preparation of Oil Phase

80 mg, 20 mg, 14 mg, and 20 mg of respective L-α-dipalmitoylphosphatidylcholine, a compound A, cholesterol, and DSPE-PEG were measured so as to make a molar ratio of 61/15/20/4, and 1.0 mL of chloroform was added thereto and was dissolved to obtain an oil phase.

Formation of Lipid Membrane

The oil phase obtained in the above-described steps was placed in an eggplant flask and ethanol and an ethyl acetate were distilled off using an evaporator to obtain a lipid membrane.

Preparation of Nucleic Acid-Holding Lipid Particle

0.25 mL of an aqueous solution of nucleic acids, which had been obtained by dissolving 5 mg of siRNA to be described below in 0.263 mL of sterilized water, was added to the above-described eggplant flask, and 1.0 mL of sterilized water were further added thereto. The mixture was hydrated while being stirred using a Vortex mixer at 55°C. The obtained solution was graded by being passed through a 0.4 μm filter using an extruder (Mini Extruder manufactured by Avanti Lipids Polar, Inc.) to obtain lipid particles holding nucleic acids.
siRNAs having the following sequences were used.

5'-GUUCAGACCACUUCAGCUU-3' (sense chain) (SEQ ID No: 1)
3'-CAAGUCUGGUGAAGUCGAA-5' (antisense chain) (SEQ ID No: 2)

Measurement of Particle Diameter of Lipid particles

The particle diameters of lipid particles of a lipid particle dispersion liquid were measured as in a state of an undiluted liquid using a zeta potential · potassium measurement system ELS-Z2 manufactured by OTSUKA ELECTRONICS Co., LTD.

Evaluation of Inclusion Rate of siRNA

(Quantitative Determination of Total Nucleic Acid Concentration)

0.01 mL of a 3M ammonium acetate aqueous solution and 0.003 mL of glycogen were added to 0.02 mL of lipid particles holding nucleic acids. Next, lipids were dissolved by adding 0.5 mL of ethanol thereto, and only nucleic acids were precipitated. After allowing the resultant to stand for two hours at -20°C, the resultant was centrifuged for 15 minutes under the conditions of 14000 x g and 4°C, and a supernatant was removed. After air-drying the resultant for 15 minutes or longer, water was added to the resultant to re-dissolve the resultant. Then, the total nucleic acid concentration was quantitatively determined by measuring the concentration of the resultant using NANODROP NF1000 (Thermo Fisher Scientific Inc.).

(Quantitative Determination of Nucleic acid Concentration in Outer Water Phase)

Quantitative determination was performed based on low-range assay disclosed in Manual and Protocol, using Quant-iT RiboGreen RNA Assay Kit (Invitrogen). First, a 20x TE buffer contained in the above-described kit was diluted with water to obtain a 1x TE buffer. A 20 times diluted solution was adjusted by adding 0.095 mL of the 1x TE buffer to 0.005 mL of lipid particles holding nucleic acids, in order to quantitatively determine only the nucleic acids of an outer water phase. Next, a sample which was diluted by 4000 times using a TE buffer was obtained without destroying lipid particles holding nucleic acids, by adding 1.99 mL of the 1 x TE buffer to 0.1 mL of the 20 times diluted solution.
1.791 mL of the 1x TE buffer was added to 0.009 mL of an undiluted RiboGreen reagent liquid to adjust a 200 times diluted solution. Subsequently, 1.98 mL of 1x TE buffer was added to 0.22 mL of the 200 times diluted solution to obtain a RiboGreen reagent diluted by 2000 times.
0.1 mL of a sample diluted by 4000 times was placed in a 96-well plate, 0.1 mL of the RiboGreen reagent which had been diluted by 2000 times was added to the sample. Then, the nucleic acid concentration in an outer water phase was quantitatively determined by measuring fluorescence (at an exciting wavelength of 485 nm and a fluorescent wavelength of 535 nm) using a plate leader Infinit EF200 (TECAN).

(Calculation of Inclusion Rate)

The inclusion rates of lipid particles holding nucleic acids in Examples 1 to 3 and Comparative Examples 1 to 3 were calculated in accordance with the following equation using the quantitative determination results of the total nucleic acid concentration and the concentration in an outer water phase which have been obtained in the above-described step.

Inclusion rate (%) = (total nucleic acid concentration – nucleic acid concentration in outer water phase) / total nucleic acid concentration x 100

The results are shown in Table 1.

[Table 1]

| | Oil phase solvent | Adjusting method | Compound A | Particle diameter (nm) | Inclusion rate (%) |
|---|---|---|---|---|---|
| Example 1 | Ethanol/ethyl acetate | Coacervation | None | 172 | 68 |
| Example 2 | Ethanol/ethyl acetate | Coacervation | Present | 324 | 94 |
| Example 3 | Ethanol/ethyl acetate | Coacervation | Present | 274 | 88 |
| Comparative Example 1 | Ethanol/ethyl acetate | Bangham | Present | 304 | 27 |
| Comparative Example 2 | Ethanol | Coacervation | Present | 222 | 54 |
| Comparative Example 3 | Chloroform | Bangham | None | 110 | 2 |

As shown in Table 1, it was found that the inclusion rate of lipid particles which had been adjusted through the coacervation method was unexpectedly improved compared to the Bangham method which is an adjusting method in the related art, even without using the compound having at least one amino group and at least one imidazoyl group. In addition, it was found that the lipid particles which hold nucleic acids and were obtained through the coacervation method using the compound having at least one amino group and at least one imidazoyl group as in Examples 2 and 3 had significantly high inclusion rate.

## Claims

1. A method for producing lipid particles including nucleic acids, the method comprising the following steps of (1) to (4):

   (1) a step of heating an oil phase containing a phospholipid, ethanol, and ethyl acetate;
   (2) a step of mixing an oil phase prepared in step (1) with a water phase containing nucleic acids, wherein the mass ratio of mixing the water phase with the oil phase is 1.6:1.0 to 1.1:1.0;
   (3) a step of cooling the mixed liquid which contains the oil phase and the water phase and has been obtained in step (2), and crystallizing lipid particles; and
   (4) a step of removing ethanol and ethyl acetate from the mixed liquid which contains the oil phase and the water phase and has been obtained in step (3).

2. The method for producing lipid particles according to claim 1,
   wherein, in step (1), the oil phase further contains a compound having at least one amino group and at least one imidazoyl group.

3. The method for producing lipid particles according to claim 2,
   wherein the compound having at least one amino group and at least one imidazoyl group is a compound represented by General Formula (1),

   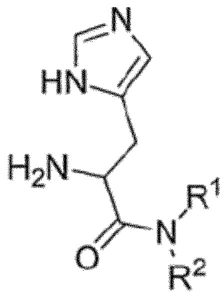

   (1)

   in the formula, $R^1$ and $R^2$ are the same as or different from each other and are substituents selected from an alkyl group having 10 to 22 carbon atoms, an alkyloxyalkylene group having 10 to 22 carbon atoms, an alkanoyloxyalkylene group having 10 to 22 carbon atoms, and an alkyloxycarbonylalkylene group having 10 to 22 carbon atoms.

4. The method for producing lipid particles according to any one of claims 1 to 3,
   wherein, in step (1), the oil phase containing a phospholipid, ethanol, and ethyl acetate is heated at 40°C to 70°C.

5. The method for producing lipid particles according to any one of claims 1 to 4,
   wherein, in step (3), the mixed liquid containing an oil phase and a water phase is cooled at 10°C to 30°C.

## Patentansprüche

1. Verfahren zum Herstellen von Nukleinsäure einschließenden Lipidpartikeln, wobei das Verfahren die folgenden Schritte von (1) bis (4) umfasst:

   (1) einen Schritt des Erhitzens einer Ölphase, welche ein Phospholipid, Ethanol und Ethylacetat enthält;
   (2) einen Schritt des Mischens einer in Schritt (1) vorbereiteten Ölphase mit einer Nukleinsäure enthaltenden wässrigen Phase, wobei das Masseverhältnis des Mischens der wässrigen Phase mit der Ölphase 1,6:1,0 bis 1,1:1,0 beträgt;
   (3) einen Schritt des Abkühlens der gemischten Flüssigkeit, welche die Ölphase und die wässrige Phase enthält und in Schritt (2) erhalten wurde, und des Kristallisierens von Lipidpartikeln; und
   (4) einen Schritt des Entfernens von Ethanol und Ethylacetat aus der gemischten Flüssigkeit, welche die Ölphase und die wässrige Phase enthält und in Schritt (3) erhalten wurde.

2. Verfahren zum Herstellen von Lipidpartikeln gemäß Anspruch 1, wobei in Schritt (1) die Ölphase weiterhin eine Verbindung mit wenigstens einer Aminogruppe und wenigstens einer Imidazoylgruppe enthält.

3. Verfahren zum Herstellen von Lipidpartikeln gemäß Anspruch 2, wobei die Verbindung mit wenigstens einer Aminogruppe und wenigstens einer Imidazoylgruppe eine durch die Allgemeine Formel (1) dargestellte Verbindung ist:

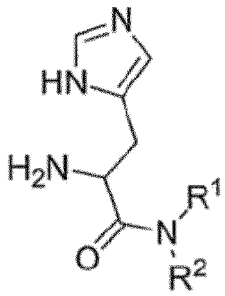

(1)

wobei in der Formel $R^1$ und $R^2$ gleich oder unterschiedlich voneinander sind und Substituenten sind, ausgewählt aus einer Alkylgruppe mit 10 bis 22 Kohlenstoffatomen, einer Alkoxyalkylengruppe mit 10 bis 22 Kohlenstoffatomen, einer Alkanoyloxyalkylengruppe mit 10 bis 22 Kohlenstoffatomen und einer Alkyloxycarbonylalkylengruppe mit 10 bis 22 Kohlenstoffatomen.

4. Verfahren zum Herstellen von Lipidpartikeln gemäß einem der Ansprüche 1 bis 3, wobei in Schritt (1) die Ölphase, welche ein Phospholipid, Ethanol und Ethylacetat enthält, bei 40°C bis 70°C erhitzt wird.

5. Verfahren zum Herstellen von Lipidpartikeln gemäß einem der Ansprüche 1 bis 4, wobei in Schritt (3) die gemischte Flüssigkeit, welche eine Ölphase und eine wässrige Phase enthält, bei 10°C bis 30°C abgekühlt wird.

## Revendications

1. Procédé pour produire des particules de lipide incluant des acides nucléiques, le procédé comprenant les étapes suivantes de (1) à (4) :

(1) une étape de chauffage d’une phase huileuse contenant un phospholipide, de l’éthanol et de l’acétate d’éthyle ;
(2) une étape de mélange d’une phase huileuse préparée dans l’étape (1) avec une phase aqueuse contenant des acides nucléiques, dans laquelle le rapport de masse du mélange de la phase aqueuse avec la phase huileuse est 1,6:1,0 à 1,1:1,0 ;
(3) une étape de refroidissement du liquide mélangé qui contient la phase huileuse et la phase aqueuse et qui a été obtenu dans l’étape (2), et de cristallisation de particules de lipide ; et
(4) une étape d’enlèvement d’éthanol et d’acétate d’éthyle depuis le liquide mélangé qui contient la phase huileuse et la phase aqueuse et qui a été obtenu dans l’étape (3).

2. Procédé pour produire des particules de lipide selon la revendication 1,
dans lequel, dans l’étape (1), la phase huileuse contient en outre un composé ayant au moins un groupe amino et au moins un groupe imidazoyle.

3. Procédé pour produire des particules de lipide selon la revendication 2,
dans lequel le composé ayant au moins un groupe amino et au moins un groupe imidazoyle est un composé représenté par la Formule Générale (1),

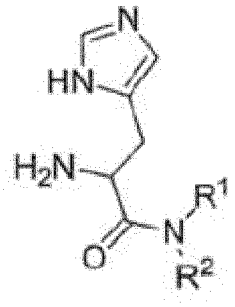

(1)

dans la formule, $R^1$ et $R^2$ sont les mêmes ou différents l'un de l'autre et sont des substituants sélectionnés à partir d'un groupe alkyle ayant 10 à 22 atomes de carbone, un groupe alkyloxyalkylène ayant 10 à 22 atomes de carbone, un groupe alcanoyloxyalkylène ayant 10 à 22 atomes de carbone et un groupe alkyloxycarbonylalkylène ayant 10 à 22 atomes de carbone.

4. Procédé pour produire des particules de lipide selon l'une quelconque des revendications 1 à 3, dans lequel, dans l'étape (1), la phase huileuse contenant un phospholipide, de l'éthanol et de l'acétate d'éthyle est chauffée à 40 °C à 70 °C.

5. Procédé pour produire des particules de lipide selon l'une quelconque des revendications 1 à 4, dans lequel, dans l'étape (3), le liquide mélangé contenant une phase huileuse et une phase aqueuse est refroidi à 10 °C à 30 °C.

FIG. 1

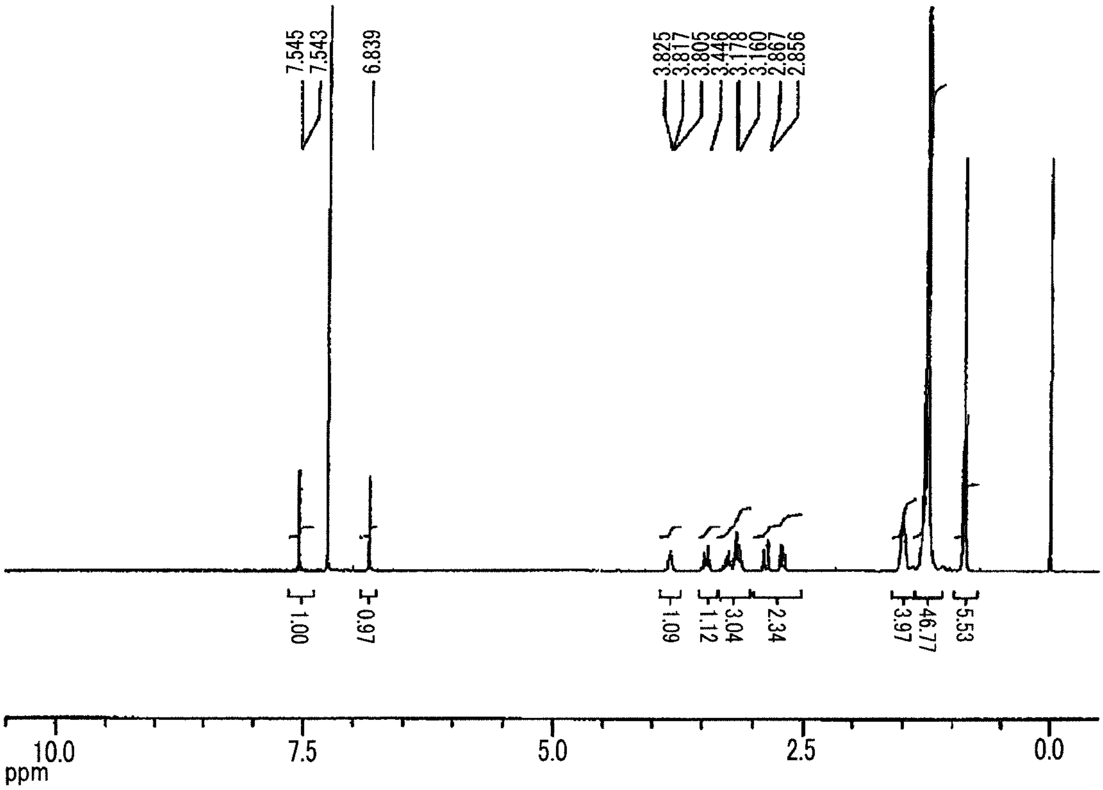
7.545
7.543
6.839
3.825
3.817
3.805
3.446
3.178
3.160
2.867
2.856
1.00
0.97
1.09
1.12
3.04
2.34
3.97
46.77
5.53
10.0
7.5
5.0
2.5
0.0
ppm

FIG. 2

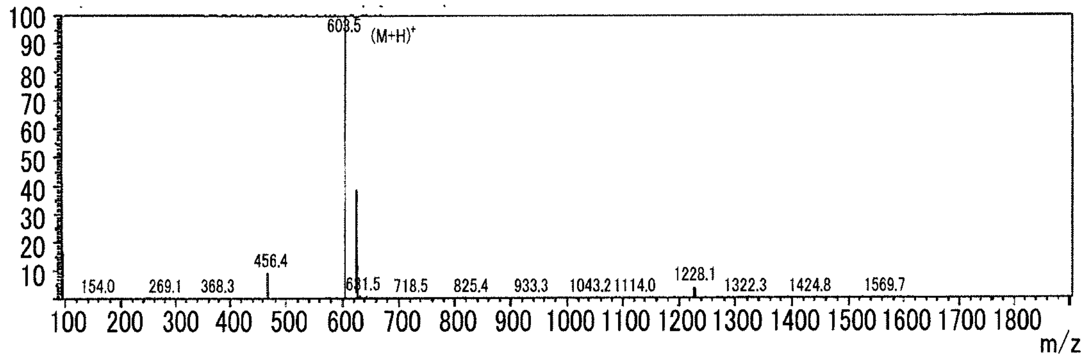
603.5
(M+H)+
456.4
154.0
269.1
368.3
631.5
718.5
825.4
933.3
1043.2
1114.0
1228.1
1322.3
1424.8
1569.7
m/z

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011021026 A
- JP 2005517739 A
- US 20120114831 A1


### Non-patent literature cited in the description


- *Gene Therapy,* 1999, vol. 6, 271
- **W. GREENE et al.** Protective Groups in Organic Synthesis. John Wiley & Sons, INC, 2007, 255-265

SEQUENCE LISTING

<110> FUJIFILM Corporation
<120> A method for production of lipid particle and a nucleic acid delivery carrier containing lipid particle
<130> 15F00616
<160> 2
<210> 1
<211> 19
<212> RNA
<213> Artificial Sequence
<220>
<223> Description of Artificial Sequence: siRNA
<400> 1
guucagacca cuucagcuu 19
<210> 2
<211> 19
<212> RNA
<213> Artificial Sequence
<220>
<223> Description of Artificial Sequence: siRNA
<400> 2
caagucuggu gaagucgaa 19